Europäisches Patentamt

(19) **European Patent Office**

**Office européen des brevets**

(11) Publication number: **0 059 221**

**A1**

(12) **EUROPEAN PATENT APPLICATION**
published in accordance with Art. 158(3) EPC

(21) Application number: **81902452.2**

(51) Int. Cl.³: **A 61 K 31/725**

(22) Date of filing: **01.09.81**

Data of the international application taken as a basis:

(86) International application number:
**PCT/JP81/00211**

(87) International publication number:
**WO82/00762 (18.03.82 82/08)**

(30) Priority: **03.09.80 JP 122563/80**
**03.09.80 JP 122564/80**

(43) Date of publication of application:
**08.09.82 Bulletin 82/36**

(84) Designated Contracting States:
**FR**

(71) Applicant: **KYOSEI PHARMACEUTICAL CO. LTD.**
**25-18, Okusawa 1-chome Otaru-shi**
**Hokkaido 047(JP)**

(72) Inventor: **DAIGO, Koji**
**4-12, Takeshirodai 4-cho**
**Sakai-shi Osaka 590-01(JP)**

(74) Representative: **Ores, Irène et al,**
**CABINET ORES 6, Avenue de Messine**
**F-75008 - Paris(FR)**

(54) **AGENT FOR ALIMENTARY CANAL.**

(57) An agent for the alimentary canal characterized by containing a water-soluble salt of alginic acid as an active ingredient.

EP 0 059 221 A1

MEDICINAL COMPOSITION FOR GASTROINTESTINAL TRACT

Technical Field and Disclosure of the Invention

The present invention relates to medicinal compositions for the gastrointestinal tract, and more particularly to medicinal compositions which have activity to protect the mucous membrane of the tract and hemostatic activity on the tract and which exhibit useful therapeutic effects on peptic ulcers such as gastric ulcer and duodenal ulcer, hemorrhagic gastric erosion (gastritis), reflux esophagitis, radiation injuries, central ulcers such as Cushing's ulcer, curling's ulcer and like stress ulcers, hemorrhage due to gastric biopsy, etc.

The term "ulcers" refer to erosion or defects on the surface of the structures of organs. They arise from a wide variety of causes which still remain to be fully clarified. So-called peptic ulcers, such as gastric ulcer and duodenal ulcer, are thought to be attributable to the growth of attacking factors (the digestive ability of the gastric juice) and the deterioration of protective factors (acting against the former). The therapeutic agents heretofore known for such ulcers include drugs for inhibiting the attacking factor, such as antichlolinergic drugs, antipepsin drugs and like antacids, and drugs reinforcing the protective factor, such as vitamin U, L-glutamine, gefarnate and slcoseryl.

The radiation therapy employed for the cancers of the esophagus, stomach, womb, tongue, etc. causes acute or chronic (tardive) inflammation or injuries in the esophagus, rectum, mouth, etc., such as esophagitis, esophageal ulcer, rectitis, erosion of the tongue, etc., due to the exposure to radiation. Effective methods of curing these diseases have not been developed except for some simple procedures of treatment, such as washing with bactericidal agents, injection of steroid and administration of gels of synthetic aluminum silicate, aluminum hydroxide and like mucous membrane protective agents. Such treatment is unable to achieve a high therapeutic effect.

Central peptic bleeding attendant on serious cerebral injuries such as cerebral tumor, cerebral vascular disorder and injuries in the head, is widely known as Cushing's ulcer. Cushing's ulcer, as well as curling's ulcer due to burning, is presently considered to be typical of stress ulcers, and many experimental and clinical findings have been accumulated. Usual stress ulcers are relatively easy to cure although frequently recurrent and seldom involve large hemorrhages that would lead to a lethal result although entailing hematemesis or hematochezia, whereas Cushing's ulcer is difficult to prevent, involves bleeding which, if occurring, is severe and difficult to check, and is therefore markedly serious, for example, in respect of mortality and one of the most formidable complications in neurosurgery. The mortality due to bleeding is said to be as high as 70 % although varying from reporter to reporter. While various preventive and therapeutic methods have been used for Cushing's ulcer, a very few of them have proved effective. The drugs and therapies which are said to be useful for usual stress ulcers are almost ineffective on Cushing's ulcer, and transfusion appears to be the only measures to be taken. Moreover the ulcer causes a very large hemorrhage, so that it is not infrequently required to replace the whole blood in the body in one day. The large amount of transfusion entails the hazard of complications.

Fiberscopes, especially those for gastic biopsy, are presently indispensable to the diagnosis of gastric diseases. Gastric biopsy is today performed by more or less forcibly removing a piece of tissues with a forceps and inevitably causes bleeding from the site of examination. The bleeding usually ceases spontaneously, but if the site includes a lesion of gastric cancer or ulcer, atrophic gastrisis or gastric polyp, bleeding occurs at a relatively high rate and is difficult to check, frequently resulting in hematemesis or hematschezia after the biopsy. To stop the bleeding due to gastric biopsy, it is practiced to locally apply an adrenaline solution or intramuscularly or intravenously administer a hemostatic agent, such as phytonadione (vitamin $K_1$), hemocoaglase (lipitinase) or the like, but it is doubtful whether or not the bleeding can be checked completely. It is desired to provide hemostatic agent and methods for effectively and reliably stopping bleeding promptly.

An object of the present invention is to provide a novel ulcer curing composition or medicinal composition for the gastrointestinal tract which contains as its active component a compound entirely different from those contained in the foregoing known agents for curing peptic ulcers.

Another object of the invention is to provide a clinically very useful novel medicinal composition which acts rapidly on radiation injuries which are not curable effectively by any of known methods to greatly alleviate subjective symptoms and satisfactorily reduce erosive changes on the mucous membrane subjectively, the composition further being capable of remarkably decreasing the frequency of central gastric tract bleeding due to Cushing's ulcer, etc. and being substantially free of side effects.

Another object of the invention is to provide a novel hemostatic composition for checking hemorrhage due to gastric biopsy.

The above objects of the invention can be fulfilled by providing a medicinal composition which is characterized in that the composition contains a water-soluble salt of alginic acid as its active component. Thus the present invention provides a medicinal composition for the gastrointestinal which is characterized by a water-soluble salt of alginic acid contained therein as its active component and which has activity to protect the mucous membrane of the gastrointestinal tract and to check bleeding from the tract.

Examples of water-soluble salts of alginic acid which are useful as active components of this invention are sodium salt, potassium salt, ammonium salt, amine salt, etc. of alginic acid. Alginic acid is a polysaccharide (composed of D-mannuronic acid and L-guluronic acid) which is a polyuronic acid present in the form of calcium salts or magnesium salts in the cell walls and between the cells of Phaeophyceae, such as Laminaria, Ecklonia and Eisenia. The acid is usually obtained by extraction from Phaeophyceae with a dilute alkali solution and purification of the extract in the form of a sodium salt. In the field of pharmaceuticals, sodium alginate is conventionally used chiefly as a material for preparations and also as an expansible laxative and a plasma extender. Since the compound has activity to cure wounds and injuries and to check bleeding, it is known also as a physical hemostatic agent (generally for topical application to injuries). However, nothing whatever is reported about the use of sodium alginate for curing peptic ulcers or about the cases wherein sodium alginate is used for curing radiation injuries and central ulcers such as curling's ulcer. Furthermore the compound has never been used as a hemostatic agent for checking bleeding due to gastric biopsy.

Moreover, sodium alginate is a natural high-molecular-weight polysaccharide and is therefore not uniform in its quality. The pharmacological activity thereof will naturally vary due to variations, for example, in its composition and molecular weight distribution, and also in viscosity when it is in the form of an aqueous solution. No research has been made on the relation between the pharmacological activity and such physicochemical properties.

We have conducted intensive research on the relation between the quality of sodium alginate and other water-soluble salts of alginic acid and the pharmacological activity thereof and found in the course of the research that these water-soluble salts of alginic acid have activity to protect the mucous membrane of the gastrointestinal tract and activity to check bleeding from the tract which activities have not been known before. Stated more specifically, we have found that an aqueous solution of sodium alginate, when given to patients with gastric ulcer, forms in the site of gastric hemorrhage or erosion insoluble alginic acid due to the participation of gastric juice and that the acid covers the site to check the bleeding, provide protection against external attacking factors and further inhibit breakdown of the tissues, whereby the lesion can be cured.

We have further found that the water-soluble salts of alginic acid produce remarkable clinical effects when used for curing radiation injuries and ulcers associated with the central nervous system and also that the salts are capable of reliably and rapidly checking the bleeding resulting from gastric biopsy. The present invention has been accomplished based on these novel findings.

The medicinal composition of this invention for the gastrointestinal tract is essentially in the form of an aqueous solution prepared by dissolving a water-soluble salt of alginic acid, serving as its active component, in water (purified water, or sterilized purified water). Useful water-soluble salts of alginic acid are usually about 60,000 to 250,000, preferably 100,000 to 200,000, in average molecular weight. The average molecular weight is determined by dissolving the alginate in 0.1 N sodium chloride, measuring the velocity of flow of the solution per second by Ostwald's viscometer and calculating the intrinsic viscosity. The 2 W/V % aqueous solution of a water-soluble salt of alginic acid having the above-mentioned average molecular weight usually has a viscosity of about 5 to 150 centipoises as measured by a B-type viscometer (two rotors, 2 min., 20 $^{o}$C). Alginates which are in this range of viscosity are suitably used in this invention to produce the desired effect. The medicinal composition of this invention for the gastrointestinal tract, which is in the form of such aqueous solution, is used usually as adjusted to a concentration of at least about 2 W/V %, preferably about 3 to 5 W/V %. When having a concentration in this range, the aqueous solution usually has a pH of about 7 and is favorably used. When desired, however, the pH is adjustable with use of a usual pH adjusting agent, such as sodium hydroxide or hydrochloric acid. Accordingly all the hydroxyl groups of the alginate to be used need not be neutralized (100 % neutralization), but at least about 50 % thereof may be neutralized. To adjust the pH to the range of 6 to 8 which is favorable for use, it is desirable to neutralize at least about 70 % of the groups. Disinfectants, flavoring agents, sweeteners and other various additives which are usually used can be incorporated into the medicinal composition of this invention by usual methods. The composition in the form of an aqueous solution, like usual liquid preparations, etc., is sterilized by heating in the usual manner.

The medicinal composition of the invention for the gastrointestinal tract is administered usually in the form of an aqueous solution having a concentration in the foregoing range. The dosage is suitably determined in accordance with the concentration of the composition, the symptoms of the patient, etc. For usual gastric ulcer, duodenal ulcer or the like, for example, the composition is given at a dose of about 10 to 100 ml, preferably about 20 to 80 ml, once daily per patient. For esophageal ulcer, it is similarly given at a dose of about 10 to 60 ml, preferably about 20 to 30 ml. In the case of radiation injuries or central ulcer, the composition is given orally at a dose of about 10 to 100 ml, preferably 20 to 80 ml, once daily per person or intrarectally at a dose of about 20 to 200 ml, preferably 40 to 160 ml, once daily per person. The time and frequency of administration are determined as desired. The composition is given at least once, preferably three to five times, daily before meals, before meals and before retiring, or between meals before the appearance of symptoms. The frequency of administration can be increased further. The method of administration is determined in accordance with the symptoms of the patient, the dose of the composition and the concentration of the active compound. Preferably the composition is given orally, via the nose or endoscopically. For application to the lower intestinal tract for colitis, rectitis, etc., the composition may be injected into the intestine.

To check the bleeding due to gastric biopsy, the composition is applied to the site of biopsy orally, or preferably endoscopically. The dose is usually about 5 to 100 ml, preferably about 10 to 60 ml, at one time but can of course be increased with the rate of bleeding from the site. The frequency of application can be increased.

The medicinal composition of this invention thus prepared and used for the gastrointestinal tract has a very low toxicity and little or no absorbing ability, assures outstanding safety, does not cause side effects or contraindications, such as thirst, visual disorder or dysuria, and exhibits the desired activity to protect the mucous membrane of the gastrointestinal tract and to check bleeding from the tract. Thus the composition is very useful as a drug for curing peptic ulcers, especially gastric ulcer and duodenal ulcer, and for curing hemorrhagic gastric erosion (gastritis) and reflux esophagitis. It is also very useful for curing central ulcers and radiation injuries, such as esophagitis, esophageal ulcer, rectitis, erosion of the tongue and the like which are caused by exposure to radiation.

The invention will be described in greater detail with reference to the following experimental examples.

Experimental Example 1

This experiment was carried out with use of the stomachs removed from rats to check aqueous solution of sodium alginate (hereinafter referred to as "AL") specimens having varying molecular weights, for the inhibitory effect on the liquefaction of the mucous membrane caused by an artificial gastric juice.

Experimental method

The stomach removed from a rat was cut open along the greater curvature, immersed in a 5 % AL solution and thereafter held placed in an artificial gastric juice (pH 1.2) at 37 °C for 30, 60, 120 or 180 minutes to measure the amount of free tyrosine produced by protein digestion in the artificial juice.

Table 1 below shows the result.

Table 1

| Experiment No. | Av. mol. wt. of AL specimen | Amount of free tyrosine ($\mu g/ml/kg$) |
|---|---|---|
| 1 | 240,000 | 62 |
| 2 | 180,000 | 65 |
| 3 | 140,000 | 61 |
| 4 | 110,000 | 65 |

| 5 | 90,000 | 78 |
| 6 | 60,000 | 77 |
| 7 | (control) | 121 |

Table 1 shows that AL has a much higher digestion inhibitory effect than the control; the compound protects the mucous membrane of the removed stomach, inhibits the self-liquefaction thereof and provides protection against external attacking factors (hydrochloric acid, pepsin, etc.). AL exhibits a high digestion inhibitory effect even when 60,000 to 90,000 in average molecular weight and achieves an especially high effect when having an average molecular weight of 110,000 to 240,000.

### Experimental Example 2

As in Experimental Example 1, this experiment was conducted with use of the stomachs removed from rats to check solutions of sodium alginate (AL, average molecular weight 140,000) of varying concentrations for the activity to protect the mucous membrane of the stomach.

Experimental method

The stomach removed from a rat was cut open along the greater curvature, fully immersed in an AL solution of specified concentration, then held placed in an artificial gastric juice (pH 1.2) at 37 °C for 2 hours and thereafter checked with the unaided eye for the degree of self-digestion. The result is shown in Table 2 according to the following criteria.

(-) The mucous membrane was almost free of liquefaction.

(+) The mucous membrane was swollen and partly liquefied.

(++) Marked liquefaction.

Table 2

| Experiment No. | Concn. of AL aq. soln. (W/V %) | Degree of self-digestion |
|---|---|---|
| 1 | 5 | (-) |
| 2 | 4 | (-) |
| 3 | 3 | (-) |
| 4 | 2 | (+) |

Table 2 shows that the AL aqueous solutions having a concentration of 3 to 5 W/V % exhibit an outstanding inhibitory effect on self-digestion (activity to protect the mucous membrane).

### Experimental Example 3

This experiment was conducted with use of the esophagi removed from rabbits to check sodium alginate (AL, average molecular weight 140,000) for its inhibitory effect on the digestion of the esophagus.

Experimental method

The esophagus removed from a rabbit was turned the membrane side out, and the opposite ends of the organ were tied with a thread. The esophagus was immersed in concentrated hydrochloric acid for 5 minutes, then lightly washed with saline, immersed in a 5 % AL solution and thereafter placed in an artificial gastric juice at 37 °C for 1, 2, 3 or 4 hours. Subsequently the juice was collected to measure the amount of free tyrosine therein. Table 3 shows the result.

Table 3

| Exp. No. | Time | Amount of free tyrosine ($\mu$ g/ml/g tissue weight) | |
|---|---|---|---|
| | | Control | AL |
| 1 | 1 | 24 | 7 |
| 2 | 2 | 46 | 14 |
| 3 | 3 | 66 | 21 |
| 4 | 4 | 83 | 29 |

Table 3 shows that AL effectively inhibits the digestion of the rabbit esophageal membrane.

Experimental Example 4

This experiment was conducted with use of rats in the following manner to check sodium alginate (AL, average molecular weight) for the hemostatic effect on bleeding due to gastric biopsy.

Experimental method

The rat was anesthetized with nembutal, and the stomach was withdrawn from the body by laparotomy and then cut open along the greater curvature. The gastric mucous membrane was partly collected with a probe to cause bleeding. Immediately thereafter, AL only, or AL and the gastric juice of the rat (1:1 in volume ratio), or AL and an artificial gastric juice (0.06 N hydrochloric acid) (1:1 in volume ratio) was applied dropwise to the site of hemorrhage to determine the duration of bleeding with the unaided eye. The above procedure was repeated three times with use of different rats. Table 4 shows the result.

Table 4

| Exp. No. | Specimen | Time taken for checking hemorrhage (sec.) | | |
|----------|----------|------|------|------|
| 1 | Control | 60 | 75 | 75 |
| 2 | AL | 60 | 75 | 75 |
| 3 | AL + rat gastric juice | 45 | 30 | 45 |
| 4 | AL + artificial juice | 30 | 45 | - |

Table 4 reveals that AL forms a gel in the presence of the gastric juice of the rat or artificial gastric juice to check the bleeding in a shortened period of time.

Experimental Example 5

The procedure of Experimental Example 4 was repeated with the exception of using specimens of sodium alginate ranging from 60,000 to 240,000 in average molecular weight in combination with the gastric juice of rat in the volume ratio of 1:1. Table 5 below shows the result.

Table 5

| Exp. No. | Av. mol. wt. of sodium alginate | Time taken for checking hemorrhage (sec.) |
|----------|---------------------------------|-------------------------------------------|
| 1 | 240,000 | 35 |
| 2 | 180,000 | 40 |
| 3 | 140,000 | 35 |
| 4 | 110,000 | 40 |
| 5 | 90,000 | 45 |
| 6 | 60,000 | 50 |
| 7 | - (control) | 90 |

Table 5 reveals that the sodium alginate specimens ranging from 60,000 to 240,000 in average · molecular weight are almost equivalent in the hemostatic effect achieved.

We have further found that sodium alginate interacts with fibrinogen which participates in the blood clotting system to promote the thrombin-induced conversion of fibrinogen into fibrin, consequently promoting clotting of the blood released to biochemically check bleeding. This effect will be apparent from the experiments to follow.

### Experimental Example 6

This experiment was conducted to check whether or not specimens of sodium alginate (AL) varying in average molecular weight as listed below interact with fibrinogen.

Experimental method

To 1.5 ml of 0.5 % solution of fibrinogen was added 1.5 ml of 1 % solution of an AL specimen of specified molecular weight, and the precipitation resulting in about 3 minutes at 25 °C was determined in terms of turbidity. Table 6 below shows the result.

Table 6

| Exp. No. | Av. mol. wt. of AL | Tubidity (650 mμ) |
|---|---|---|
| 1 | 240,000 | 0.732 |
| 2 | 200,000 | 0.755 |
| 3 | 180,000 | 0.747 |
| 4 | 160,000 | 0.760 |
| 5 | 140,000 | 0.749 |

| 6 | 110,000 | 0.735 |
| 7 | 90,000 | 0.747 |
| 8 | 60,000 | 0.710 |
| 9 | 40,000 | 0.264 |
| 10 | - (Control) | 0.0 |

Table 6 shows that sodium alginate (AL) has ability to precipitate fibrinogen from the fibrinogen solution. This is suggestive of the bonding of fibrinogen with AL through hydrogen bond, hydrophobic interaction and analogous force, whereby the AL increases the effective concentration of fibrinogen to contribute to the clotting of the blood at the site of bleeding.

### Experimental Example 7

This experiment was carried out in the following manner by adding thrombin to fibrinogen solution to check whether or not sodium alginate (AL, average molecular weight 140,000) influences the formation of fibrin.

Experimental method

(1)　　To 0.4 ml of 0.2 % fibrinogen solution was added 0.4 ml of an AL solution of specified concentration, and 0.1 ml (0.1 unit) of a thrombin solution was added to measure the fibrin clotting time. The experiment was performed at 28 °C. Table 7 below shows the result.

Table 7

| Exp. No. | AL concn. (W/V %) | Fibrin clotting time |
|---|---|---|
| 1 | 5 | 1 min. 50 sec. |
| 2 | 4 | 2 min. 00 sec. |
| 3 | 3 | 2 min. 15 sec. |

| 4 | 2 | 2 min. 30 sec. |
| 5 | 1 | 3 min. 00 sec. |
| 6 | 0 (No AL) | 6 min. 00 sec. |

Table 7 reveals that AL promotes clotting at a concentration of 1 to 5 W/V %. Although the compound of course acts effectively even when used at concentrations of above 5 W/V %, the viscosity of the aqueous solution increases with the increase of the concentration, presenting difficulties in administration and the insertion of the probe at too high a concentration to cause a trouble, hence undesirable.

Given below are examples wherein medicinal compositions are prepared according to the invention and clinical examples wherein such compositions were used.

Preparation Example 1

Sodium alginate having an average molecular weight of about 140,000 is placed into cold purified water in an amount of about 10 times the amount of the alginate and is allowed to stand overnight for swelling. Subsequently the compound is added to an amount of purified water so that the resulting solution will have a final concentration of 5 W/V %. The mixture is stirred to thoroughly dissolve the sodium alginate. The sodium alginate aqueous solution thus obtained is heated at 100 °C for 30 minutes for sterilization and thereafter placed into containers to obtain a medicinal composition of the invention in the form of an aqueous solution.

### Preparation Example 2

An aqueous solution of sodium alginate is prepared in the same manner as in Preparation Example 1 except that sterilized purified water is used in place of purified water. The solution is placed into containers to obtain a medicinal composition of the invention.

### Clinical Example 1

Cases

Thirteen cases of gastric ulcer and 7 cases of hemorrhagic gastric erosion. Table 1 shows the age distribution of the patients.

Table 1 Age Distribution

| Age | Patient | | |
|---|---|---|---|
| | Total | Male | Female |
| ---19 | 0 | 0 | 0 |
| 20- 29 | 0 | 0 | 0 |
| 30- 39 | 4 | 3 | 1 |
| 40- 49 | 6 | 3 | 3 |
| 50- 59 | 3 | 2 | 1 |
| 60- | 7 | 5 | 2 |
| | 20 | 13 | 7 |

The numerical values in the parentheses are percentages as is the case with the tables to follow.

How and when to administer

The medicinal composition of the invention prepared in Preparation Example 1 above was orally given four times a day before every meal and before retiring, at a dose of 50 ml each time, for 30 days.

Results

Tables 2-1 and 2-2 show the effects produced on subjective symptoms in 30 days. The gastric ulcer and hemorrhagic gastric erosion were found to have disappeared 100 % within 30 days.

Table 3 shows the effects as evaluated endoscopically. The composition acted effectively on both gastric ulcer and homorrhagic gastric erosion in respect of hemostatic effect, occult blood test result, curing effect and subjective symptoms without any exception.

Table 2-1 Effects on Subjective Symptoms
(gastric ulcer)

| Symptoms | Total | Number of cases of disappearance in 30 days (%) |
|---|---|---|
| Epigastric pain | 13 | 13 (100) |
| Epigastralgea discomfort | 11 | 11 (100) |
| Flatus | 13 | 13 (100) |
| Sense of retention | 13 | 13 (100) |
| Heartburn | 13 | 13 (100) |
| Eructation | 13 | 13 (100) |
| Anorexia | 13 | 13 (100) |
| Nausea | 11 | 11 (100) |
| Vomiting | 6 | 6 (100) |
| Hematemesis | 2 | 2 (100) |
| Hematschezia | 6 | 6 (100) |
| Blush and pallor | 3 | 3 (100) |
| Prostration | 3 | 3 (100) |
| Palpitation | 1 | 1 (100) |

### Table 2-2 Effects on Subjective Symptoms
### (hemorrhagic gastric erosion)

| Symptoms | Total | Number of cases of disappearance in 30 days (%) |
|---|---|---|
| Epigastric pain | 7 | 7 (100) |
| Epigastralgea discomfort | 7 | 7 (100) |
| Flatus | 6 | 6 (100) |
| Sense of retention | 7 | 7 (100) |
| Heartburn | 7 | 7 (100) |
| Eructation | 7 | 7 (100) |
| Anorexia | 7 | 7 (100) |
| Nausea | 7 | 7 (100) |

### Table 3 Evaluation of Effects on Diseases

| | Effect | Criteria | | | |
|---|---|---|---|---|---|
| | | Very effective | Effective | Slightly effective | Not effective |
| Gastric ulcer | Hemostatic | 1 (7.7) | 10 (76.9) | 2 (15.4) | 0 (0) |
| | Occult blood test | 5 (55.6) | 3 (33.3) | 1 (11.1) | 0 (0) |
| | Curing | 1 (7.7) | 10 (76.9) | 2 (15.4) | 0 (0) |
| | Subjective symptoms | 1 (7.7) | 10 (76.9) | 2 (15.4) | 0 (0) |
| Hemorrhagic gastric erosion | Hemostatic | 0 (0) | 7 (100) | 0 (0) | 0 (0) |
| | Curing | 0 (0) | 7 (100) | 0 (0) | 0 (0) |
| | Subjective symptoms | 0 (0) | 7 (100) | 0 (0) | 0 (0) |

Discussion

The aqueous solution of sodium alginate was used for 13 cases of gastric ulcer and 7 cases of hemorrhagic gastric erosion to check the solution for its effectiveness. The solution was found effective in all the cases of hemorrhagic gastric erosion and also proved effective in almost all cases of gastric ulcer. The preparation was especially effective for checking large hemorrhages.

The administration of the present composition entailed no side effect whatever.

### Clinical Example 2

The medicinal composition of the invention used was the 5 W/V % aqueous solution of sodium alginate, 140,000 in average molecular weight, prepared in Preparation Example 1.

Cases and method of administration

The composition was given four times a day before every meal and before retiring, at a dose of 25 ml each time, for 5 cases of gastric ulcer, 2 cases of duodenal ulcer, 1 case of hemorrhagic gastritis and 2 cases of reflux esophagitis. The composition was administered for 5 days to 150 days. Table 4 shows the age distribution of the patients.

Table 4

| Age | Total | Patient | |
| --- | --- | --- | --- |
| | | Male | Female |
| - 19 | 0 | 0 | 0 |
| 20 - 29 | 0 | 0 | 0 |

| | | | |
|---|---|---|---|
| 30 - 39 | 1 | 1 | 0 |
| 40 - 49 | 1 | 1 | 0 |
| 50 - 59 | 4 | 3 | 1 |
| 60 - | 4 | 1 | 3 |
| Total | 10 (100) | 6 (60) | 4 (40) |

Results

Table 5-1 to Table 5-4 show the results achieved in respect of subjective symptoms. The symptoms of hemorrhagic gastritis subsided within 30 days in all cases. The subjective symptoms of reflux esophagitis, gastric ulcer and duodenal ulcer disappeared in about 21 days.

Table 5-1 Disappearance of Subjective Symptoms
(gastric ulcer)

| Symptoms | Total | Disappearance of symptoms within: | | | |
|---|---|---|---|---|---|
| | | 5 days | 10 days | 21 days | No effect |
| Chest pain | 2 | 2 | | | |
| Epigastric pain | 5 | | | 3 | 2 |
| Esophageal pain | 3 | 1 | 1 | 1 | |
| Allotrylic sense in esophagus | 2 | | 2 | | |
| Abdominal pain | 5 | | | 1 | 4 |
| Epigastralgea discomfort | 5 | | 2 | 1 | 2 |
| Flatus | 5 | | 2 | 1 | 2 |
| Sense of retention | 4 | 1 | 3 | | |
| Heartburn | 4 | 2 | 2 | | |
| Anorexia | 3 | | | 3 | |
| Nausea | 5 | 1 | 1 | 3 | |

| | | | | |
|---|---|---|---|---|
| Vomiting | 3 | 1 | 2 | |
| Reflux | 2 | 1 | 1 | |
| Hematemesis | 2 | 1 | | 1 |
| Hematschezia | 2 | 1 | | 1 |
| Stricture | 1 | | 1 | |
| Blush and pallor | 1 | | | |
| Prostration | 1 | | 1 | |

Table 5-2 Disappearance of Subjective Symptoms
(duodenal ulcer)

| Symptoms | Total | Disappearance of symptoms within: | | | |
|---|---|---|---|---|---|
| | | 5 days | 10 days | 21 days | No effect |
| Epigastric pain | 2 | | | | 2 |
| Esophageal pain | 1 | | | | 1 |
| Allotrylic sense in esophagus | 2 | | | | 2 |
| Abdominal pain | 1 | | | | 1 |
| Epigastralgea discomfort | 2 | | 1 | | 1 |
| Flatus | 1 | | 1 | | |
| Sence of retention | 2 | 1 | 1 | | |
| Heartburn | 2 | 1 | 1 | | |
| Eructation | 2 | 1 | 1 | | |
| Anorexia | 1 | 1 | | | |
| Nausea | 2 | | 1 | | 1 |
| Vomiting | 1 | | | | 1 |
| Reflux | 1 | 1 | | | |
| Hematschezia | 1 | | 1 | | |

Table 5-3 Disappearance of Subjective Symptoms
(hemorrhagic gastritis)

| Symptoms | Total | Disappearance of symptoms within: | | | |
|---|---|---|---|---|---|
| | | 5 days | 10 days | 21 days | 30 days |
| Sense of retention | 1 | | | | 1 |
| Heartburn | 1 | | | | 1 |
| Anorexia | 1 | | | | 1 |

Table 5-4 Disappearance of Subjective Symptoms
(reflux esophagitis)

| Symptoms | Total | Disappearance of symptoms within: | | | |
|---|---|---|---|---|---|
| | | 5 days | 10 days | 21 days | No effect |
| Chest pain | 1 | 1 | | | |
| Esophageal pain | 2 | 1 | | | 1 |
| Allotrylic sense in esophagus | 2 | 1 | | | 1 |
| Epigastralgea discomfort | 1 | 1 | | | |
| Heartburn | 2 | 1 | | | 1 |
| Eructation | 1 | 1 | | | |
| Anorexia | 1 | | | | 1 |

Table 6 shows the effects of the composition as evaluated based on the results obtained endoscopically in 30 days.

Table 6 Effects of the medicinal composition

| | Effect | Very effective | Effective | Slightly effective | Not effective |
|---|---|---|---|---|---|
| | | | Criteria | | |
| Gastric ulcer (5 cases) | Hemostatic | 1 | 3 | 1 | 0 |
| | Curing | 3 | 2 | 0 | 0 |
| | Subjective symptoms | 3 | 2 | 0 | 0 |
| Duodenal ulcer (2 cases) | Hemostatic | 0 | 1 | 1 | 0 |
| | Curing | 0 | 1 | 1 | 0 |
| | Subjective symptoms | 0 | 2 | 0 | 0 |
| Hemorrhagic gastritis (1 case) | Hemostatic | 0 | 1 | 0 | 0 |
| | Curing | 0 | 1 | 0 | 0 |
| | Subjective symptoms | 0 | 0 | 1 | 0 |
| Reflux esophagitis (2 cases) | Hemostatic | 0 | 1 | 1 | 0 |
| | Curing | 1 | 1 | 0 | 0 |
| | Subjective symptoms | 0 | 2 | 0 | 0 |

Table 6 reveals the following.

(1) Effects on gastric ulcer

The hemostatic activity was very effective in one case out of five (20 %), effective in three cases (60 %) and slightly effective in one case (20 %), thus achieving 80 % of effectiveness with the very effective and effective results combined. The ulcer disappeared very effectively in three cases (60 %) and effectively in two cases (40 %), thus attaining 100 % effectiveness with these very effective and effective results combined. The composition similarly achieved 100 % effectiveness on the subjective symptoms. Consequently the composition proved very useful in all the cases.

(2) Effects on duodenal ulcer

Bleeding was checked and the ulcer disappeared effectively in one case out of two (50 %) and slightly effectively in one case (50 %), hence 50 % effectiveness. The subjective symptoms subsided effectively in the two cases. Thus these two cases substantiate the usefulness of the composition.

(3) Hemorrhagic gastritis

The composition was effective in respect of hemostatic activity and disappearance of the ulcer and slightly effective on the subjective symptoms, hence useful.

(4) Effects on reflux esophagitis accomplished by esophageal hiatus hernia

The hemostatic activity was effective in one case out of two (50 %) and slightly effective in one case (50 %) to achieve 50 % effectiveness. The ulcer disappeared very effectively in one case (50 %) and effectively in one case (50 %), hence 50 % effectiveness. The composition was effective on the subjective symptoms in both cases and therefore useful in these cases.

Clinical Example 3 Radiation Esophagitis

While receiving treatment on cancer, the patients of the following two cases with erosion of the esophagus were orally given the composition prepared in Preparation Example 1 three times a day before every meal at a dose of 40 ml each time and were checked for changes in the subjective symptoms with the lapse of time and observed endoscopically.

Patient 1 : 68-year-old male with esophagus cancer

The patient received radiation therapy for cancer of esophagus in the midportion of the esophagus in the chest region. About the time he had been irradiated with 3000 rad, he experienced a burning sensation in the bone portion of the rear chest and a swallowing pain. An endoscopic examination at about this time revealed a highly hemorrhagic erosive inflammation over a length of about 8 cm above a stricture due to cancerous ulcer. The subjective symptoms were remarkably alleviated several days after the start of administration of the composition. In two weeks after the start of administration, hardly any erosive inflammation was endoscopically found, with reduced susceptibility to bleeding.

Patient 2 : 56-year-old female with lung cancer

While receiving radiation therapy for cancer in an upper portion of the right lung, the patient had a sensation of a lump or tightness and a swallowing pain in an upper esophageal region to a lower throat portion included in the radiation field. The subjective symptoms were lessened by the administration of the composition for two days and almost disappeared on and after the 10th day.

The administration of the composition was continued for 32 days and thereafter discontinued. Subjective symptoms did not appear for more than one month thereafter. An X-ray observation revealed no abnormality in the esophageal mucous membrane in the corresponding region even during the appearance of symptoms. Presumably the inflammation, if any, was mild. No endoscopic examination was performed because the site in question was not easily accessible.

Clinical Example 4 Rectitis due to Radiation

The patients of the following two cases with rectitis resulting from the treatment for cancer of the womb were treated by intrarectally injecting the present composition once daily at a dose of 100 ml and allowing the patient to stay in bed for about 1 hour after the injection. The results are given below.

Patient 3 : 62-year-old female with cancer of womb

The patient received radiation therapy for uterine cancer 4 years ago, and radiation rectitis was thereafter found by X-ray with improper expansion of the rectum. She complained of a lower abdominal pain during defecation, with the result of occult blood test remaining positive. Administration of the composition eliminated hematschezia within 1 week, also eliminating or alleviating the abdominal pain and occult blood. The expansion insufficiency of the rectum was found to remain unchanged by X-ray.

Patient 4 : Cancer of womb

The patient continually experienced a small amount of bleeding from the anus upon every defecation. after receiving radiation therapy for cancer of the womb five years ago. In the second week after the start of administration of the composition, the anal hemorrhage apparently lessened, with defecation occasionally accompanied by no bleeding.

Clinical Example 5 Erosion of Tongue by Radiation

The patient was instructed to gargle 4 to 5 times daily with 30 ml of the present composition each time.

Patient : 54-year-old male with cancer of tongue

The patient was treated by irradiation of radiation (60 Co) and a radium needle for a cancerous tumor in the left half of the back of the tongue. Two weeks later, an erosion and a fur occurred on the needle-pierced portion. With the present composition administered for 1 week, the erosion on the back of the tongue was cured completely, and the attendant pain disappeared. Thus the erosion on the needle-pierced portion was cured much more promptly than is generally the case with such erosions.

Clinical Example 6 Tardive Ulcer at Distal End of Esophagus

In the following two cases, the present composition was given orally three times daily before every meal at a dose of 40 ml each time for a shallow ulcer at the distal end of the esophagus. The ulcer appeared to have developed tardively due to irradiation with radiation during gastrectomy.

Patient 6 : 77-year-old male with gastric cancer

One year and 6 months ago, the patient received radiation therapy during gastrecomy performed for gastric cancer. For 1 month before coming to the hospital, he experienced a sensation of retention at a lower portion of the esophagus during swallowing. X-ray and endoscopic examination revealed a shallow linear ulcer in the esophageal mucous membrane immediately above the pylorus. Passage of barium was greatly impeded by the leison. In the third week after the start of administration of the present composition, X-ray studies revealed a remarkable improvement in the passage of barium. In the fifth week, the ulcer was found to have been cured endoscopically.

Patient 7 : 68-year-old male with gastric cancer

As in the above case (patient 6), he received radiation therapy during gastrecomy performed for gastric cancer 2 years ago. For several months before coming to the hospital, he experienced a penetrating pain and a sensation of slight retention at a lower portion of the esophagus during swallowing. Small but many lesions of shallow erosive ulcer were endoscopically noted at the distal end of the esophagus. A continued treatment was performed with oral administration of an antacid and usual mucous membrane protective agent such as aluminum hydroxide gel, but the subjective symptoms as well as endoscopic findings remained unchanged without any improvement.
However, the pain disappeared with administration of the
present composition for 1 week, and the sensation of
retention also disappeared in 3 weeks. The small erosive
ulcerous lesions were found to have been disappeared and
no inflammatory lesions were noted on the mucous membrane
when the patient was endoscopically checked in the fourth
week.

Clinical Example 7 Cushing's Ulcer

1. Cases

Table 7 below shows the original diseases involved
in 26 cases of Cushing's ulcer studied. Although not
without exceptions, patients with Cushing's ulcer as a
complication generally have serious cerebral dysfunctions
and the resulting disturbance of consciousness. Twenty-
five cases out of the 26 cases were in a semi-coma to deep
coma, i.e. at a serious level of disturbance of consciousness.
The present composition was therefore given through a tube
inserted into the stomach through the nose since oral
administration was in no way possible. Coffee-ground vomits
and tarry stools were noted in all the cases. The gastric
juice was collected through a tube from time to time and
checked by occult blood test, and the stool was also
similarly checked to detect hemorrhage, if any. The case
involving the likelihood that blood was swallowed due to an
injury in the mouth or nasal mucous membrane was of course
excluded from the present study. As a rule, the composition was given 3 times daily at a dose of 50 ml each time.

Table 7 Original Diseases of the Cases

| Disease | Number of Cases |
|---|---|
| Intracerebral hemorrhage | 12 |
| Cerebral aneurysm | 7 |
| Cerebral tumor | 5 |
| Cerebral infarction | 1 |
| External wound in head | 1 |
| Tatal | 26 |

2. Exitus

Table 8 shows the exitus of the 26 cases studied.

Table 8 Exitus of Cases

| Exitus | Death | No Change | Improvement | Cure |
|---|---|---|---|---|
| No. of cases | 9 | 3 | 10 | 4 |

Table 8 shows 9 cases of death out of the 26 cases. However, as will be described later, the hemorrhage due to Cushing's ulcer per se resulted in death in only one case. The improvement, achieved in 10 cases, means an improvement in the level of consciousness and also an improvement in neurologically abnormal symptoms. The cure, achieved in 4 cases, refers to the patient discharged without any neurological deciduate complaint. Thus 14 cases were improvement or cure. On the other hand, 3 cases remained unchanged. These results indicate that improvements in clinical symptoms can be expected in a majority of cases if Cushing's ulcer is overcome. It is therefore significant to reduce deaths from hemorrhage due to Cushing's ulcer.

3. Discussion of the Cases of Death

Table 9 shows the causes of death in the 9 cases.

Table 9

| Causes | Number of Cases |
|---|---|
| Hemorrhage due to Cushing's ulcer | 1 |
| Acute renal insufficiency | 1 |
| Numbness of brain stem due to serious cerebral dysfunction | 7 |

Table 9 shows that the hemorrhage death due to Cushing's ulcer itself is limited to only one case out of nine. Almost all deaths resulted from numbness of the brain stem due to the original cerebral dysfunction. In view of the fact that in the case of Cushing's ulcer, the hemorrhage from the ulcer itself usually entails many deaths, the present composition apparently acts against Cushing's ulcer effectively. Five cases out of the 9 cases of death were examined by autopsy. Table 10 shows the findings obtained by examining the stomach and duodenum with the unaided eye by autopsy.

Table 10 Findings obtained by Autopsy

| Case | Age | Sex | Original diesease | Results of Observation with Unaided Eye |
|---|---|---|---|---|
| 1 | 55 | M | Cerebral tumor | Slight erosion |
| 2 | 60 | F | Cerebral aneurysm | Almost no change |
| 3 | 48 | M | Cerebral tumor | Submucosal hemorrhage at stomach upper region |
| 4 | 49 | F | Cerebral aneurysm | Two small ulcerous lesions at gastric angle and vestibulum pyliri. |

Small ulcerous lesion in duodenal bulb.

| 5 | 68 | M | Cerebral aneurysm | Was exposed from lesser curvature at stomach-esophagus border closer to pylorus. A 3-cm-long linear ulcerous lesion at stomach upper region. |

In Case 5, which appears to be the only case of hemorrhage death, a thick artery closer to the lesser curvature was exposed from the membrane in the vicinity of the border between the stomach and the esophagus. Presumably bleeding from the vessel is the cause of death. In the present case about 3-cm-long linear ulcerous lesion was noted close to the lesser curvature at a stomach upper region but was free of bleeding. The patient also had serious pneumonia and was clinically in the condition of serious ventilation insufficiency, so that some question remains as to whether or not the cause of death is hemorrhage only. The patient of Case 4 died directly of acute renal insufficiency and also had serious hepatocirrhosis. The patient had 3 small lesions of ulcer in the gastric angle to the vestibulum pyliri and a lesion of ulcer in the duodenal bulb. All the lesions were in the course of curing or free of bleeding. In the other three cases, there was clinically no gastrointestinal bleeding, and autopsy conducted failed to reveal any particular abnormality in the stomach or the duodenum.

4. Results

Table 11 shows the clinical effects achieved.

- 35 -

Table 11  Clinical Effects

| Effect | No effect | Effective | Very effective | Unknown | Total |
|--------|-----------|-----------|----------------|---------|-------|
| Number of cases | 5 | 15 | 5 | 1 | 26 |

With reference to Table 11 above, the term "no effect" refers to the case wherein no reduction in hemorrhage was noted despite the administration of the composition, and the term "effective" refers to the case wherein the hemorrhage was decreased by the administration and completely stopped within 2 days to 2 weeks. The cessation of hemorrhage was determined by the occult blood test of the gastric juice or stool upon the change from positive to negative. The term "very effective" refers to the case wherein the hemorrhage ceased completely within two days after the start of the administration. Of the 5 cases of "no effect" listed, 3 cases are death, of which Case 5 in Table 10 only appears to be hemorrhage death. Another case is death due to acute renal insufficiency (as listed in Table 10), and the patient of the other case died of cerebral ischemia resulting from contraction of the brain vas. Of two cases of survival, one is a 83-year-old patient with cerebral hemorrhage and was transferred to the internal department in a vegetable state. Hemorrhage was persistent in the other case, but the patient nevertheless was generally in good condition including consciousness and therefore underwent a surgical operation, which revealed an artery exposed from the duodenal bulb. The 26 cases include a considerably large number of effective and very effective cases combined, i.e. 20 cases. This substantiates the effectiveness of sodium alginate on Cushing's ulcer. The patient of the unknown case had cerebral hemorrhage, developed a massive gastrointestinal bleeding immediately after the onset of symptoms and was hospitalized 4 hours after the onset. Although immediately given sodium alginate, the patient died 12 hours after the onset. Therefore the case was too emergent to determine the effectiveness.

Clinical Examples 3 to 6 given above indicate that the therapeutic composition of this invention acts effectively on the acute and tardive mucosal disturbances attendant on radiation therapy, i.e. on mucosal inflammation and injuries due to radiation, alleviating subjective symptoms promptly and remarkably and also mitigating the objective symptoms of erosive changes in the mucous membrane. In the case of mucosal radiation inflammation and injuries, the disturbance of cells by radiation retards the multiplication of the cells, presenting difficulty in repairing the erosion and frequently leading to chronic symptoms, whereas it is believed that the curing composition of this invention, with its outstanding ability to adhere to the mucous membrane, effectively protects the erosive portion of the membrane to produce the remarkable clinical effects described above.

Clinical Example 7 reveals that the curing composition of the invention greatly reduces the likelihood of hemorrhage death resulting from Cushing's ulcer which has not been fully clarified, for which methods of therapy still remain to be explored despite a vast accumulation of experimental and clinical studies heretofore conducted, and which entails hemorrhages that are extremely difficult to check if taking place and result in an exceedingly high rate of mortality unlike usual stress ulcers.

### Clinical Example 8

The patient was a 31-year-old male with gastric submucosal neoplasma (fibroid). Gastric endoscopy was performed to examine a tumor detected immediately below the pyrorus by a gastric group examination. A lesion resembling submucosal neoplasma was endoscopically noted on the rear wall of the stomach immediately below the pylorus. An ulcer in $A_2$ stage was found at the top of the lesion. There was no hemorrhage from the base of the ulcer. Although bleeding was expected, a biopsy was performed on the base of the ulcer for the histological diagnosis of the gastric submucosal neoplasma. When 5 pieces of tissues were removed from the base of the ulcer, the blood flowed out. The bleeding was observed for about 3 minutes but did not cease, so that the composition prepared in Preparation Example 1 was given with use of Gastroscope GF-B3 (product of Olympus Kogaku Co., Ltd.) of the general type commercially available for biopsy. A washing polyethylene tube, available as an attachment and measuring 2.2 mm in outside diameter and 1.5 mm in inside diameter, was passed through a forceps hole, 2.8 mm in diameter, of the instrument and inserted through the ulcer to its bleeding base. A 10 ml quantity of the composition was applied to the base somewhat dropwise. Five minutes thereafter, the blood clotted in the form of cables, and the blood was no longer seen to flow out along the clot. When the cable-like blood coagulation was removed by a biopsy forceps to confirm the cessation of bleeding, bleeding occurred again although slowly. Another 10 ml quantity of the composition was therefore applied dropwise to the bleeding base of the ulcer in the same manner as above. The bleeding ceased completely 4 minutes later.

No hematemesis or tarry stool was noted after the cessation of the bleeding.

## Clinical Example 9

The present composition was applied to the patients listed in Table 12 below and undergoing a gastric biopsy to determine the hemostatic effect of the composition on the hemorrhage due to endoscopic biopsy.

Table 12

| Age | Total | Male | Female |
|---|---|---|---|
| - 19 | | | |
| 20 - 29 | 1 (100) | 0 | 1 (100) |
| 30 - 39 | 3 (100) | 2 (66.7) | 1 (33.3) |
| 40 - 49 | 4 (100) | 3 (75.0) | 1 (25.0) |
| 50 - 59 | 6 (100) | 3 (50.0) | 3 (50.0) |
| 60 - | 6 (100) | 4 (66.7) | 2 (33.3) |
| | 20 (100) | 12 (60) | 8 (40) |

Disease
Gastric ulcer        12
Gastric cancer       4
Chronic gastritis    4

The values in the parentheses are percentages.

A tube 2.4 mm in inside diameter was inserted into the stomach of the patient with use of a gastrofiberscope of the direct view type 3.2 mm in forceps diameter. Immediately after biopsy, 30 ml of the composition was endoscopically applied to the site. The result was compared with 5 cases in which the composition was not given.

For the evaluation of the hemostatic effect, the fiberscope was held in place for 10 minutes, and when the bleeding ceased within this period of time, the result was interpreted as being effective.

With reference to Table 13 below showing the hemostatic effects achieved, the hemorrhage ceased within 10 minutes in all the 4 cases of chronic gastritis (100%), in 11 cases out of 12 cases of gastric ulcer (91.7%) and in 3 cases out of 4 cases of gastric cancer (75%). Of the 5 cases of control in which the present composition was not applied after biopsy, only one case proved effective (20%) with the hemorrhage stopped within 10 minutes.

Table 13

|  | Disease | Number of Cases | Effective |
|---|---|---|---|
| Test group | Chronic gastritis | 4 (100) | 4 (100) |
| | Gastric ulcer | 12 (100) | 11 (91.7) |
| | Gastric cancer | 4 (100) | 3 (75.0) |
| Control | Chronic gastritis | 5 (100) | 1 (20.0) |

The values in the parentheses are percentages.

What is claimed is:

1. A medicinal composition for the gastrointestinal tract having activity to protect the mucous membrane of the gastrointestinal tract and to check bleeding from the tract, characterized in that the composition comprises a water-soluble salt of alginic acid as its active component.

2. A medicinal composition as defined in Claim 1 which is a composition for curing peptic ulcers.

3. A medicinal composition as defined in Claim 1 which is a composition for curing radiation injuries.

4. A medicinal composition as defined in Claim 1 which is a composition for curing central ulcers.

5. A medicinal composition as defined in Claim 1 which is a composition for curing Cushing's ulcer.

6. A medicinal composition as defined in Claim 1 which is a composition for curing curling's ulcer.

7. A medicinal composition as defined in Claim 1 which is a hemostatic composition for checking bleeding due to gastric biopsy.

8. A medicinal composition as defined in any one of Claims 1 to 7 wherein the water-soluble salt is selected from among sodium salt, potassium salt, ammonium salt and amine salt.

9. A medicinal composition as defined in any one of Claims 1 to 7 wherein the water-soluble salt has an average molecular weight of about 60,000 to 250,000.

10. A medicinal composition as defined in any one of Claims 1 to 7 wherein the water-soluble salt has a viscosity of 5 to 150 centipoises when formulated into 2 W/V % aqueous solution.

11. A medicinal composition as defined in any one of Claims 1 to 6 which is administered orally or via the nose.

12. A medicinal composition as defined in any one of Claims 1 to 7 which is administered endoscopically.

13. A medicinal composition as defined in any one of Claims 1 to 6 which is administered intraintestinally.

# INTERNATIONAL SEARCH REPORT

International Application No **PCT/JP81/00211**

0059221

## I. CLASSIFICATION OF SUBJECT MATTER (if several classification symbols apply, indicate all) [3]

According to International Patent Classification (IPC) or to both National Classification and IPC

Int. Cl.$^3$    A61K 31/725

## II. FIELDS SEARCHED

### Minimum Documentation Searched [4]

| Classification System | Classification Symbols |
|---|---|
| I P C | A61K 31/725 |

### Documentation Searched other than Minimum Documentation to the Extent that such Documents are Included in the Fields Searched [5]

Osaka-fu Byoin Yakuzaishikai-hen "Iyakuhin Yoran (Sogo Shinpan)", Yakugyo Jihosha, "Saikin no Shinyaku", Yakuji Nipposha, Vol. 11-30

## III. DOCUMENTS CONSIDERED TO BE RELEVANT [14]

| Category [*] | Citation of Document, [16] with indication, where appropriate, of the relevant passages [17] | Relevant to Claim No. [18] |
|---|---|---|
| X | "Saikin no Shinyaku", Yakuji Nipposha, Vol. 11, P. 179 | 1 - 13 |
| A | Osaka-fu Byoin Yakuzaishikai-hen "Iyakuhin Yoran (Sogo Shinpan)", Yakugyo Jihosha, P. 900-901 | 1 - 13 |

* Special categories of cited documents: [15]
"A" document defining the general state of the art
"E" earlier document but published on or after the international filing date
"L" document cited for special reason other than those referred to in the other categories
"O" document referring to an oral disclosure, use, exhibition or other means

"P" document published prior to the international filing date but on or after the priority date claimed
"T" later document published on or after the international filing date or priority date and not in conflict with the application, but cited to understand the principle or theory underlying the invention
"X" document of particular relevance

## IV. CERTIFICATION

| Date of the Actual Completion of the International Search [2] | Date of Mailing of this International Search Report [2] |
|---|---|
| November 5, 1981 (05.11.81) | November 30, 1981 (30.11.81) |

| International Searching Authority [1] | Signature of Authorized Officer [20] |
|---|---|
| Japanese Patent Office | |

Form PCT/ISA/210 (second sheet) (October 1977)